(19)

Europäisches Patentamt
European Patent Office
Office européen des brevets

(11) **EP 3 651 156 A1**

(12) **EUROPEAN PATENT APPLICATION**

(43) Date of publication:
**13.05.2020 Bulletin 2020/20**

(51) Int Cl.:
***G16B 50/20*** (2019.01)

(21) Application number: **19177466.0**

(22) Date of filing: **29.05.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.11.2018 EP 18205046**

(71) Applicant: **BioSistemika d.o.o.**
**1000 Ljubijana (SI)**

(72) Inventors:
• **Zupancic, Klemen**
**1000 Ljubljana (SI)**
• **Bernot, Nejc**
**1000 Ljubljana (SI)**

• **Pirc, Zan**
**1000 Ljubljana (SI)**
• **Lalic, Jasna**
**10290 Zapresic (HR)**
• **Gruden, Kristina**
**1000 Ljubljana (SI)**
• **Cepin, Urska**
**3202 Ljubecna (SI)**
• **Nemec, Blaz**
**5292 Rence (SI)**
• **Zupancic, Luka**
**1000 Ljubljana (SI)**

(74) Representative: **Hoefer & Partner Patentanwälte mbB**
**Pilgersheimer Straße 20**
**81543 München (DE)**

(54) **NUCLEIC ACID-BASED DATA STORAGE**

(57) Method of storing information in nucleic acid is disclosed which comprises processing units of information into permutation numbers by a reversible algorithm, providing a library of *n* distinct oligonucleotide strings of predetermined length in a fixed order, wherein *n* is a positive integer, wherein each distinct oligonucleotide string is associated with a distinct index indicating the ordinal position, assembling distinct oligonucleotide strings to create strands comprising at least two oligonucleotide strings, wherein each oligonucleotide string's ordinal position matches with a permutation number obtained in step a), wherein each strand comprises at least a data bearing part and a semantic part, wherein the semantic part is to allocate orientation and/or order to a strand.

FIG. 1

SEQUENCE LISTING

<110> BioSistemika d.o.o.

<120> Nucleic acid-based data storage

<130> BSI190501PEP

<160> 28

<170> BiSSAP 1.3.6

<210> 1
<211> 26
<212> DNA
<213> Artificial Sequence

<220>
<223> forward primer

<400> 1
gcaattgatg gcggtagagc gacaga                                        26

<210> 2
<211> 26
<212> DNA
<213> Artificial Sequence

<220>
<223> reverse primer

<400> 2
tctaccgcca tcaattgctc tgtcgc                                        26

<210> 3
<211> 26
<212> DNA
<213> Artificial Sequence

<220>
<223> forward primer

<400> 3
tctgcaagct ggagtagagc gacaga                                        26

<210> 4
<211> 26
<212> DNA
<213> Artificial Sequence

<220>
<223> reverse primer

<400> 4
tctactccag cttgcagatc tgtcgc                                        26

<210> 5
<211> 26
<212> DNA
<213> Artificial Sequence


<220>
<223> forward primer

<400> 5
taggatacgc cacacactgc gacaga                                    26


<210> 6
<211> 26
<212> DNA
<213> Artificial Sequence


<220>
<223> reverse primer

<400> 6
agtgtgtggc gtatcctatc tgtcgc                                    26


<210> 7
<211> 26
<212> DNA
<213> Artificial Sequence


<220>
<223> forward primer

<400> 7
taacgtcggc tactcacagc gacaga                                    26


<210> 8
<211> 26
<212> DNA
<213> Artificial Sequence


<220>
<223> reverse primer

<400> 8
tgtgagtagc cgacgttatc tgtcgc                                    26


<210> 9
<211> 26
<212> DNA
<213> Artificial Sequence


<220>
<223> forward primer

<400> 9

tcgtgttagt ccgtcagtgc gacaga                                  26


<210> 10
<211> 26
<212> DNA
<213> Artificial Sequence


<220>
<223> reverse primer

<400> 10
actgacggac taacacgatc tgtcgc                                  26


<210> 11
<211> 26
<212> DNA
<213> Artificial Sequence


<220>
<223> forward primer

<400> 11
aacactcgtc tcgaccatgc gacaga                                  26


<210> 12
<211> 26
<212> DNA
<213> Artificial Sequence


<220>
<223> reverse primer

<400> 12
atggtcgaga cgagtgtttc tgtcgc                                  26


<210> 13
<211> 26
<212> DNA
<213> Artificial Sequence


<220>
<223> forward primer

<400> 13
tctatgcgac cactacgagc gacaga                                  26


<210> 14
<211> 26
<212> DNA
<213> Artificial Sequence


<220>
<223> reverse primer

<400> 14
tcgtagtggt cgcatagatc tgtcgc                                                    26

<210> 15
<211> 26
<212> DNA
<213> Artificial Sequence

<220>
<223> forward primer

<400> 15
tgacagagcg tctatcgtgc gacaga                                                    26

<210> 16
<211> 26
<212> DNA
<213> Artificial Sequence

<220>
<223> reverse primer

<400> 16
acgatagacg ctctgtcatc tgtcgc                                                    26

<210> 17
<211> 26
<212> DNA
<213> Artificial Sequence

<220>
<223> forward primer

<400> 17
atggctatcg ctgattgcgc gacaga                                                    26

<210> 18
<211> 26
<212> DNA
<213> Artificial Sequence

<220>
<223> reverse primer

<400> 18
gcaatcagcg atagccattc tgtcgc                                                    26

<210> 19
<211> 26
<212> DNA
<213> Artificial Sequence

<220>
<223> forward primer

<400> 19
tcctgcgctt atcagagtgc gacaga                                              26


<210> 20
<211> 26
<212> DNA
<213> Artificial Sequence


<220>
<223> reverse primer

<400> 20
actctgataa gcgcaggatc tgtcgc                                             26


<210> 21
<211> 26
<212> DNA
<213> Artificial Sequence


<220>
<223> forward primer

<400> 21
tcgctagaag agcagagtgc gacaga                                             26


<210> 22
<211> 26
<212> DNA
<213> Artificial Sequence


<220>
<223> reverse primer

<400> 22
actctgctct tctagcgatc tgtcgc                                             26


<210> 23
<211> 26
<212> DNA
<213> Artificial Sequence


<220>
<223> forward primer

<400> 23
agtgtccagg attgcatggc gacaga                                             26


<210> 24
<211> 26
<212> DNA
<213> Artificial Sequence

<220>
<223> reverse primer

<400> 24
catgcaatcc tggacacttc tgtcgc                                              26


<210> 25
<211> 26
<212> DNA
<213> Artificial Sequence


<220>
<223> forward primer

<400> 25
accgatacac gtcgtacagc gacaga                                             26


<210> 26
<211> 26
<212> DNA
<213> Artificial Sequence


<220>
<223> reverse primer

<400> 26
tgtacgacgt gtatcggttc tgtcgc                                             26


<210> 27
<211> 26
<212> DNA
<213> Artificial Sequence


<220>
<223> forward primer

<400> 27
gtgcatgcat cacgatgagc gacaga                                             26


<210> 28
<211> 26
<212> DNA
<213> Artificial Sequence


<220>
<223> reverse primer

<400> 28
tcatcgtgat gcatgcactc tgtcgc                                             26

**Description**

**FIELD OF THE INVENTION**

The present invention is concerned with a method of storing information in nucleic acid. The method comprises converting units of information into permutation numbers which are stored in the form of sequences of nucleotides.

**BACKGROUND**

During the last several years a new era called the "data age" has arisen. The data age is characterized by the quick transition of analog to digital data, alongside with a huge increase in new data being generated on daily basis. Data has become critical to all aspects of life with the rise of internet; smart home devices and internet of things, communication and social media, autonomous cars, humanoid robots, and the like, transforming education and entertainment alike, transforming work and living environments, and the way the world is perceived. This digital existence, as defined by the sum of all data created, captured, and replicated on Earth in any given year, is called the *"global datasphere"*.

The amount of digital data in the world is exponentially growing, but ability to store all that data is not keeping pace. It is expected that by 2025 the "global datasphere" will grow to 163 zettabytes (that is a trillion gigabytes), that is ten times of data generated in 2016. Current infrastructure can handle only a fraction of the coming data deluge, which is expected to consume all of the world's microchip-grade silicon by 2040. This fundamental change gives rise to the new challenges of managing, interpreting and storing big data.

Another problem is the lifetime of the presently used standard media used for archiving data, such as optical discs, hard drives, and magnetic tapes, which is only a few years.

Therefore there is a need for an alternative storage medium, which stores data efficiently and reliably for an extended period of time.

DNA appears to be an excellent candidate for an alternative storage medium due to its enormous information capacity, extreme spatial compactness, long term stability and basically no maintenance costs. All living organisms run on the same software language: DNA. In other words DNA has proven to be a stable, robust and long-living medium.

There are two main approaches to DNA synthesis: chemical, such as using phosphoramidite synthesis and enzymatic, such as using template-free polymerase (terminal deoxynucleotidyl transferase). Usually, the former uses solid state whereas the latter is an aqueous process.

There have been several attempts to use DNA for storing data, with approaches based on chemical or on enzymatic synthesis of DNA.

Examples for an approach using chemical synthesis is semiconductor-based synthetic DNA manufacturing process featuring a high-throughput silicon platform with parallel synthesis, another one is generation of large quantities of a few different DNA molecules with up to about 30 base pairs and using combinatorial enzymatic reactions to encode information into the recombination patterns of those prefabricated bits of DNA. Instead of mapping one bit to one base pair, bits can be arranged in multidimensional matrices, and sets of molecules represent their locations in each matrix.

Examples for an approach using enzymatic synthesis of DNA is a three-step enzymatic DNA synthesis by using terminal deoxynucleotidyl transferase (TdT) and a reversible terminator.

While progress is made in developing methods for storing information into DNA there still is an urgent need for an improved, cost-efficient, and reliable method of storing information in DNA due to the increasing data creation and the consequent increasing need for data storage. There is a need of a highly reliable method that allows to encode and decode information in nucleic acids in an efficient way.

**DESCRIPTION OF THE INVENTION**

The present invention is concerned with a method of storing information in nucleic acids. The present invention provides an improved, cost-efficient, and reliable method of storing information in nucleic acids. The invention combines a novel and inventive encoding algorithm with the use of polynucleotides for storage. The present invention is based on custom combination of precast nucleotide sequences into larger assemblies which are ready to be decoded. Those assemblies comprise information in such arrangement that allows unambiguous assignment and clear allocation of the encoding/decoding order, wherein the order is identified by an included semantic information, such as mutually semantically overlapping parts or separate semantic data.

The method of the present invention and the structure of the sequences used provide high flexibility and allow to choose and adapt elements for any type of data, and for any amount of information. The present invention provides a method for storing data which is very flexible, but at the same time secure and safe. It can be adapted for use of huge and small amounts of data. In particular the method of the present invention allows to store huge amounts of data in very small place.

The method of the present invention comprises processing units of information into permutation numbers, providing a library of distinct oligonucleotides, also referred to as oligonucleotide string or string, wherein each distinct oligonucleotide is associated with an index, matching an index with a permutation number and picking an oligonucleotide string with the matched index, and assembling the picked distinct oligonucleotide strings to strands, such as braids, wherein each strand comprises at least a data bearing part and a semantic part. The semantic part can be included in one or any of the oligonucleotide strings or can be provided as separate sequence. The nucleic acid, thus, comprises data which can be decoded and semantic information which indicates the relationship between different oligonucleotides or polynucleotides, respectively.

**DEFINITIONS**

The following terms are used in this description:

"Information to be stored" can be any information that is present in the form of a code or that can be translated into a code, in particular digital data, such as binary data, such as text, data pools, databases, research data, books, pictures, movies, etc.

A "unit of information" refers to any unit comprising information to be stored.

A "data bearing sequence" is a sequence, that can be polynucleotide, an oligonucleotide, or a part of it, and that represents a number or symbol or integer to be stored. This number or symbol or integer can be translated/converted into information. In particular, a data bearing sequence represents a number that matches with a permutation number. A data bearing sequence can have a length of a few nucleotides, such as 3 to 20, or a higher number of nucleotides such as hundreds or even thousands of nucleotides.

A "data bearing unit" is a unit which comprises at least one data bearing sequence but can additionally comprise further parts like a gluing part. Examples for data bearing units are strings, ropes, and braids.

A "data system" is a group of data bearing sequences or units, such as braids, i.e. at least two braids, carrying data that can be converted into information to be stored. The order of braids, and, thus, of the data bearing sequences, within a data system is defined by the semantic part of braids.

The term "nucleic acid" refers to sequences of nucleotides, such as an oligo- or polynucleotide, or a nucleotide sequence or a base sequence or a piece of DNA, which can be small, such as comprising only a few nucleotides, for example 2 to 100 nucleotides, or can be large, such as comprising hundreds or thousands or up to millions of nucleotides. The terms "nucleotide" and "base" are used exchangably, i.e. the term "base" shall refer to a nucleotide as long as the context permits. Moreover, a nucleotide sequence can be a sequence of any type of naturally occurring or unnatural, synthetically obtained nucleotides or variants thereof, i.e. monomer units for forming a nucleic acid, and in particular a DNA sequence. When it is referred to oligonucleotides this shall refer in particular to DNA but can comprise any other nucleic acid and its modifications useful for this purpose. Examples for nucleic acids are sequences of deoxyribonucloetides, i.e. adenosine triphosphate (ATP), guanosine triphosphate (GTP), cytidine triphosphate (CTP), and thymidine triphosphate (TTP).

The term "oligonucleotide" as used in the present application refers to nucleic acids having a length of about up to 500 nucleotides, such as 2 to 80, or 4 to 60, for example 4 to 20 nucleotides. The term "oligonucleotide" is interchangeably used with the terms "oligonucleotide string" and "string". Oligonucleotides or strings of the present invention can be assembled as described in detail in this application. Assembling of oligonucleotides means that a strand or a sequence is created by combining distinct oligonucleotides.

A "strand" is a sequence comprising at least two oligonucleotide strings. A strand can comprise a few or many different oligonucleotide strings in a predetermined order. A strand also comprises a semantic part which allows to allocate orientation and/or order to the oligonucleotide strings within the strand and the order of the strand within a data unit. The semantic part can be part of a data bearing oligonucleotide or can be included in specific oligonucleotides that are part of the strand.

A "library" comprises distinct nucleotide sequences each of which can be identified by an index, such as a reference number, or a semantic part. The index can be any type of character or combination of characters which can be used to identify distinct objects, such as numbers, letters, symbols or a combination thereof.

A "string" or oligonucleotide string is one type of data bearing unit, that is an oligonucleotide or a piece of DNA or a nucleotide sequence that comprises a data bearing sequence and optionally a semantic and/or gluing part. Strings are oligonucleotides that can have the function of a primer and can be combined to form assemblies, like ropes and/or braids. The length of a string can be from a few bases such as three or four bases up to hundreds or thousands bases. A string can be single-stranded (ss-string) or double-stranded (ds-string). The term string comprises single and double stranded sequences.

A "string library" is a library of groups of strings, wherein any string has the same number of bases and wherein in a group each string has the same sequence and is associated with the same index, and strings of different groups have different sequences and different indices, wherein a string of one group cannot have the same sequence as a string of another group.

The term "rope" refers to a construct that has been obtained by combining two strings and, thus, comprises two data bearing units such as strings and optionally a gluing part. A gluing part, if present, can be at one end, on both ends or between two strings. A rope can be single-stranded (ss-rope) or double-stranded (ds-rope). The term rope comprises single and double stranded sequences. As strings are associated with an index, a rope comprising two strings is associated with the indices of these strings in a defined order.

A "rope library" is a library of groups of ropes, wherein in a group each rope has the same data bearing sequences, and wherein ropes of different groups are different. A rope library can be produced by assembling two distinct strings and amplifying the ropes obtained.

In one embodiment a library of ropes can be produced from two sets of strings, wherein both sets have the same number of distinct strings and wherein ropes can be created as Cartesian products of the two sets of strings.

The term "braid" refers to a construct that has been obtained by combining data bearing units like strings and/or ropes and optionally semantic parts and/or gluing parts. In other words, a braid comprises at least two data bearing sequences, in particular at least three data bearing sequences, such as at least two or three strings. The number of data bearing units in a braid is variable, it can be only a few such as three to six, or medium size, such as 10 to 500, or big size, such as 500 to 5000, or mega size, such as 5000 to many thousand, for example up to 5 million. The length of a braid and/or the one or more sets of data bearing units used to build it is defined by the amount of information which can be stored in a braid. The braid is built from data bearing units, such as strings and/or ropes, and it can optionally comprise one or more gluing parts. A braid can have a specific structure as defined below. In general it is composed of one or more data bearing or coding parts and one or more semantic or order defining parts. A coding part comprises at least one data bearing sequence.

A "semantic part" serves for defining the order and/or orientation of braids but can additionally also bear information. A semantic part can be part of a string or rope, i.e. a string or rope can comprise at least one semantic part, or it can be separate, for example a separate sequence that is added to a braid. A semantic part can also be formed by the structure or order of strings, such as head-tail pairs.

The term "index" when used in the present application refers to ordinal data for defining the order of oligonucleotides, the term index can include ordinal and categorical data, for example an index can include combinations of numbers, of numbers and letters, of numbers and/or letters and/or symbols, etc.

"Mutually semantically overlapping parts" are parts within a braid, such as a head oligo and a tail oligo, that can be used to clearly allocate the orientation and/or order of braids, in other words the order of sequences within different braids.

A "gluing part" is a sequence that does not contribute information but forms an overlapping part, such as an overlapping end to allow combining two or more sequences such as strings or ropes, for example to combine two or three strings in a predetermined order to form a rope, or to combine ropes for creating a braid. The gluing part can have any length that allows combining, such as about 3 to about 80 bases. The gluing part can have any sequence that allows assembling and does not interfere with the data bearing part. Gluing parts of different strings can be different or equal, preferably the gluing sequence is the same for all strings.

A "reversible algorithm" is an algorithm which can be used to code and decode data. Any type of algorithm which can be used for processing digital data and results in integers and vice-versa is comprised by this term. The algorithm can be symmetric or asymmetric, for example can use open source algorithm in one direction, confidential algorithm in the other direction.

The term "partial permutation" refers to the number of choices available for ordering r distinct objects from a finite set of *n* different objects without repetition.

The term "offset" is used for the number of possible partial permutations of all subsequent spots.

The term "permutation number" refers to a positive number or integer which is obtained by processing an information code using partial permutation enumeration, i.e. a number of choices available for a position of an object, such as a position in a series of strings.

The term "matching" refers to the relationship between a permutation number and an index. In other words the indices of oligonucleotides are enumerated on a list in a predetermined fixed order such that every index is associated with a number which corresponds to a permutation number.

The method of the present invention allows to store information by assembling oligonucleotide strings to create a nucleic acid strand which comprises a data bearing part and a semantic part in a very efficient, resource-saving way. The method of the present invention is as defined in the claims.

The method of the present invention comprises that units of information, which are present as digital data, usually as binary code, are processed using partial permutation enumeration to obtain permutation numbers which are matched with indices of oligonucleotide strings in a library of distinct oligonucleotide strings. Those strings that have an integer matching with the permutation number are then assembled to strands in an order according to the information by using a semantic part which allocates orientation and order of oligonucleotide strings in the strand. The strands can be stored, amplified and decoded in an easy manner. By decoding the information is available again. It is the gist of the present invention to densify information by processing data and using oligonucleotides for "representation" of integers that are connected with the unit of information by an algorithm.

Usually information is provided in the form of or can be converted to binary codes. Thus, usually a binary code is the starting material. Methods of converting information such as text, images, databases, data pools, research data, books, movies etc., into binary code are well-known in the art. A text for example can be converted into a binary ASCII code with 7 bit-per-letter encoding resulting in a binary sequence.

For processing the starting code in step a), usually a binary code, is chopped into smaller pieces. The size of the pieces is not critical, it depends on the availability of distinct strings. The pieces can all have the same size or can have different sizes. The number of oligonucleotide strings available corresponds to or is related to the number of permutations available, among others. Before or when processing the pieces of the binary code it can be read as integer in a code such as a decimal code or a code to another base, such as hexadecimal. Conversion of data from digital or binary to decimal or hexadecimal code is well-known and can be done automatically.

It is necessary that the algorithm is reversible, so that the conversion can be done in both directions. An algorithm can be chosen depending on the amount of data to be stored and dependent on other considerations, for example confidentiality of information. The method of the invention is explained for a method wherein binary data are chopped to smaller pieces and then are read as decimal integers. The method is similar when using data on other base. The integers are processed and permutation numbers are created for every piece of information. The permutation number is matched with the index of an oligonucleotide of a library.

Processing occurs by defining an offset for each position of a series of strings in a braid and by dividing the integer by the offsets, wherein the offset is defined as the number of options available for a position in a partial permutation as outlined in more detail below. Processing provides permutation numbers which are matched with the indices of oligonucleotides and are stored as nucleotide sequences as explained in more detail below. The data carried by the nucleotide sequences, for example in the form of braids, can be decoded to receive the coded information in the form of permutation numbers which can be converted back to the binary code.

The medium for storing are nucleic acids, in particular oligonucleotides the length of which can be adapted according to the amount of information to be stored. Therefore, in step b) of the method of the present invention, a library is provided which comprises a plurality of distinct objects which are associated with an index. The distinct objects are oligonucleotides of predetermined length, comprising a data bearing sequence and optionally a semantic part and/or a gluing part. The oligonucleotides can be assembled to strands that can be stored as data unit. Assembling the oligonucleotides which carry the necessary information can be done in different ways and approaches are outlined below to create and amplify libraries of oligonucleotides and to prepare data systems.

Examples for a library to be used in step b) are a string library or a rope library, i.e .a library comprising a number of strings, wherein all strings preferably are of identical length, or a library comprising a number of ropes, wherein ropes are oligonucleotides comprising two strings.

In one embodiment a library of oligonucleotides, which are called "strings", is provided wherein each string is associated with an index which index can be matched with a permutation number. The library can comprise single-stranded or double-stranded oligonucleotides, usually it comprises single-stranded strings. The strings are assembled to provide braids wherein the braids carry the information about integers in a specific order and semantic data. To obtain braids, strings can be assembled or strings can be combined to build ropes as an intermediate step and ropes can then be assembled to provide braids.

The strings each comprise a unique nucleotide sequence. Each type of string is provided in a separate vessel, each string or vessel, respectively, is associated with an index. The number of distinct oligonucleotides is defined as being $n$, wherein $n$ is an integer. For example the index can be an integer from 0 to n-1, each index being assigned to one type of strings. The value of $n$ corresponds to the number of available distinct oligonucleotides which is dependent on the number of nucleotides in a string and also on other parameters as outlined below. The more distinct oligonucleotide strings are available, the more indices are available which can be allocated to permutation numbers and the more information can be stored in a data unit.

Thus, more information can be stored with a higher number of distinct oligonucleotides, which higher number is available with longer sequences. The number of distinct oligonucleotides can also define the number of braids available for one data unit. For example, when the approach with head and tail pairs of braids is used, and when the number of distinct oligonucleotides is $n$, the number of available braids is n-1. When the approach with braids carrying indices as semantic part is used, the number of available braids is much higher. Thus, the length of the strings, and the length and number of braids can be chosen depending on the amount of information to be stored in a data system. This allows high flexibility for the system and adaptation to the needs.

As an example, if strings have 4 nucleotides, 16 distinct strings are possible and because of restrictions 14 different strings are available in the library; the strings will be assigned an index starting with 0 and going up to 13. Thus, each string can be associated with an ordinal number - the index, and encodes this information. This information can be decoded as outlined above and examples are shown below and in the examples.

It has been found that oligonucleotide strings to be used for preparing strands according to the present invention preferably should fulfil the following conditions to overcome errors in the sequences and to provide for safe and reliable information:

a) sufficient Levenshtein or Hamming distance between each pair of strings, making it possible to detect and correct errors, for example at least 3, such as at least 4 or at least 5;
b) restricting GC content, for example to a range between about 0.35 and about 0.75, such as 0.4 to 0.6, preferably between 0.5 and 0.55;
c) defining the number of G/C bases in the last part of the string, for example not more than 3, such as not more than 2 in the last 8 to 5 bases;
d) restricting the number of identical bases in a row, for example only up to 3, such as up to 2, identical bases in a row, and/or restricting the number of repeats in a sequence, for example to no more than 3, such as no more than 2 of such repeats in a sequence.

As strings can be used as primers in a PCR, it is useful when the sequence of the strings complies with requirements for primers as is known in the art.

It has been found that good results are obtained when the G/C content is between 0.35 and 0.75, such as 0.4 to 0.6, for for example between 0.5 and 0.55. The length of a sequence of an oligonucleotide can for example be in a range of about 3 to about 500 nucleotides, such as about 6 to about 100 nucleotides, for example 8 to 20 nucleotides. As outlined before the number of nucleotides defines the number of permutations and, thus, the amount of information to be stored. The smaller the oligonucleotides the more stable are the strings but the fewer the number of permutations. The higher the number of nucleotides in a string the higher the number of permutations but the more complex and costly the system. The optimal combination can be found by a skilled person using routine experimentation.

Furthermore, it has been found that good results are obtained when the number of G/C bases that are present within the last 5 to 10 bases of each string is restricted, for example to not more than 3, such not more than 2, such as not more than 2 G/C bases in the last 5 bases. Furthermore, it has been found that it is useful to restrict the number of identical bases in a row, for example such that there are not more than three or not more than two of 2-base repeats are present in each string. This avoids problems with secondary structures that may disturb the assembly, such as hairpins or loops.

Furthermore the use Levenshtein or Hamming distance between each pair of coding sequences should be sufficient to detect and correct errors. The Levenshtein or Hamming distance can be at least 3, for example at least 4, preferably at least 5. A Levenshtein or Hamming distance of at least 5 means that at least 5 single base changes are required to turn one coding sequence into another coding sequence. If there are no more than 2 errors, the resulting sequence will still be closer to the correct original sequence than to any other of the coding sequences.

In one embodiment of the method of the present invention, in step b) in a preliminary reaction strings are combined to form ropes, which ropes then can be used to create braids. In this embodiment, for example a series of first amplification reactions can be carried out to obtain ropes comprising two strings. Each rope comprises two unique string sequences which can be separated by a gluing sequence. For example, a first string can comprise a gluing sequence and can be used as a primer in an amplification reaction, a second string sequence can be added so that a double-stranded rope is obtained by extending the first string using the complementary and inverse sequence of a second coding sequence as a template.

Thus, a rope can for example be obtained by annealing a forward primer of a first pair of single-stranded oligonucleotides to a reverse primer of second pair of single-stranded oligonucleotides, extending both the reverse and forward primers by PCR to obtain the double-stranded oligonucleotide rope. One strand of the rope comprises the coding sequence of the forward primer of the first pair at its 5' end, and the coding sequence of the forward primer of the second pair at its 3' end. In this embodiment the gluing sequence will be between the two coding sequences, i.e the strings. The ropes, thus, provide pre-created combinations of strings that can be used for creating braids in step c), wherein the ropes are selected based on the order determined in step a).

If a gluing sequence is used, the length can vary from about 4 to about 80 nucleotides, such as about 6 to about 60 nucleotides. The optimal length can be found by a skilled artisan depending for example on the length of the string. A length of about 6 to about 16 bases, such as 8 to 10 bases has been found useful.

A library of ropes can be provided for example by using a method wherein two strings are assembled in an intermediate amplification step resulting in ropes comprising two unique coding sequences. For preparing this library of ropes, two sets of data bearing units - strings, wherein both sets have the same number of distinct oligonucleotides of predetermined length, can be used. Ropes can be created as Cartesian products of the two sets to provide a plurality of ropes which can then be used to be assembled to braids. As an example, ropes can be prepared as pairs of single-stranded oligonucleotides, wherein each pair comprises a forward and a reverse primer, wherein the forward primer comprises a first single-stranded oligonucleotide comprising a first coding sequence at its 5' end and a first or unique gluing sequence at its 3' end, and wherein the reverse primer comprises a second coding sequence at its 5' end and a second or unique gluing sequence at its 3' end, wherein the second coding sequence is complementary and inverse to the first coding sequence, and wherein the second gluing sequence is complementary and inverse to the first gluing sequence, and wherein the gluing sequence in each pair of single-stranded oligonucleotides is identical, and wherein each coding sequence is unique.

In a further embodiment a library of ropes is provided, with ropes without a gluing sequence. The ropes, each comprising two unique sequences derived from two single-stranded strings, can be used for preparing braids.

These single-stranded oligonucleotide ropes are similar to the ropes as defined above, but do not carry a gluing sequence.

Ropes of a library are associated with an index or indices, respectively, indicating for each rope the ordinal position of each string within each rope. These types of oligonucleotides comprising two pre-combined strings allow to assemble braids in one PCR amplification step instead of two PCR amplification steps by pooling the single-stranded oligonucleotides each comprising two strings and amplifying the mixture of ropes to obtain braids as described below.

To obtain a data unit of the present invention, strings and/or ropes comprised in a library, that can be prepared as outlined above, are assembled in the order that has been determined by an algorithm according to step a).

Step c) of the method of the present invention for storing information comprises assembly of data units, by allocating or matching oligonucleotides via their indices to permutation numbers and picking and combining those oligonucleotides in a correct order. Thus, nucleic acids, comprising data-bearing and optionally semantic data, are assembled from smaller units, i.e. strings or ropes, to strands, such as braids, in a very efficient, resource-saving way. The gist of the invention is that oligonucleotides can be used to carry information, wherein the information is not comprised directly in the sequence, but by allocation to an index used for storing information. Thus, it is not necessary to prepare oligonucleotides depending on the information to be stored, but a library of oligonucleotides can be used for many different purposes.

To assemble strings any method that allows to connect to oligonucleotides can be used. Some examples are outlined in the following. For the method of the present invention it is important that the strings in a braid are in a specific order and that the order of the braids can be determined when decoding the braid. In other words, both the order of the strings and the order of the braids is important.

To decode the information of a data unit it is necessary to know the order of the braids and, thus the position of each braid in a row, i.e. if a braid is the first one, the second one, the $n^{th}$ one or the terminal one. To provide this information each braid comprises a semantic part. The semantic part can be an index type part, i.e. provide an index for each braid which indicates the order of the braids. Another option is to provide a structure for the braids of a data unit that allows this allocation. As one example, in the braids, the heads and tails can provide the information about the "position" of a braid, when the head string of a braid is identical to the tail string of the preceding braid, and starting string and terminal string are unique. For this embodiment of a data unit, there will be one unique starting string, pairs of head and tail strings, where each pair can be used only once per data unit, and one unique terminal string which can be used only once in a data unit. Furthermore, the braid can comprise a number of center strings, wherein each center string can be used only once per braid.

The strings or ropes forming the braids of the present invention can be assembled by any known method for assembling oligonucleotides. For example, the strings or ropes can be assembled via enzymatic DNA synthesis using at least part of the single-stranded oligonucleotides as primers. This specific method has the advantage that it is "green technology" avoiding or minimizing the use of chemical agents.

There are many options for assembling strings and/or ropes. An important issue is that orientation and order of the strings in a strand or braid, respectively, are correct and that orientation and order are derivable and the algorithm is reversible. This is provided by the assembly and the use of a semantic part. To provide the correct assembly and access to the semantic data different options are available. In one embodiment, gluing parts are used to assemble strings or ropes in the right order. Another option is, to combine/ligate two strings to ropes without gluing and provide a library of ropes wherein the assembly occurs via overlapping parts in each rope, as is outlined in detail below.

If for example a braid is assembled from six strings with indices matching with permutation numbers as described above, the following concepts can be used. It is assumed that strings $C_1$, $C_2$, $C_3$, $C_4$, $C_5$, $C_6$ have been chosen from a library comprising $n$ distinct strings, each string having an index. The strings have been chosen such that their indices match with permutation numbers in the correct order, as determined according to step a).

Two examples are outlined for assembly of strings $C_1$ to $C_6$ to a braid. In a first approach $C_1$ and $C_2$ are annealed via a gluing part, elongated and amplified to give rope $R_1$, comprising coding sequences of $C_1$ and $C_2$. In the same way strings $C_2$ and $C_3$, $C_3$ and $C_4$, $C_4$ and $C_5$, and $C_5$ and $C_6$ are annealed, elongated and amplified to give ropes $R_2$, $R_3$,

$R_4$, and $R_5$. Each of the ropes overlaps with the following rope in the coding part of one string. All ropes are mixed in about equivalent volume amounts and subjected to a PCR wherein the overlapping parts provide for connection. Thus, a braid which comprises strings $C_1$ to $C_6$ is obtained. In some cases it might be useful to use terminal ropes in excess.

In another approach ropes are formed without a gluing part. For example, a library of ropes is provided by producing Cartesian products of two coding sets of strings wherein the ropes can be identified by indices which indicate the combination of strings per rope. Thus, a library of ropes is provided which comprises ropes that each consist of a combination of two strings.

To assemble a braid, ropes are picked which each comprise two strings as required for the braid in the right order. The strings in each rope provide for an overlapping part with the precedent or following rope as follows. In one embodiment braids are obtained by annealing ropes, wherein any rope has an overlapping part with another rope, wherein the overlapping part is a forward primer or a reverse primer, respectively, such that one string comprises a forward primer and a second string comprises a corresponding reverse primer. When such ropes are annealed forward primers of a single-stranded rope and corresponding reverse primers of other single-stranded oligonucleotides are combined to obtain a double-stranded braid.

As an example if a braid shall be assembled with strings (which are at the same time strings and primers) $SP_1$, $SP_2$, $SP_3$, $SP_4$, $SP_5$, $SP_6$, the following single-stranded ropes are used: The first rope comprises $SP_1$, which will provide either the unique starting string of the data unit or the head string of the braid, and additionally $SP_2$ as forward string/primer. The second rope comprises $SP_2$ and $SP_3$ as reverse string/primers, the third rope comprises $SP_3$ and $SP_4$ as forward string/primers, the fourth rope comprises reverse string/primers $SP_4$ and $SP_5$ and the fifth rope comprises forward string/primers $SP_5$ and $SP_6$, wherein $SP_6$ is the tail string or the terminal string, respectively. When these ropes are mixed and amplified in a single step with end primers in excess, a braid is obtained wherein strings $SP_1$ ... $SP_6$ are in the correct order in a polynucleotide sequence.

Since PCR requires both forward and reverse primers, this would mean that for each combination of two strings two copies would be required. If two sets of coding sequences are used instead, the number of possible permutations can be increased without increasing the number of raw materials required, as all forward primers would for example be composed of $C_a$-$C_b$, while all reverse primers will be composed of $C_b$-$C_a$, i.e. ropes for all center strings. In this approach, only for the head string forward and reverse primers would be necessary.

Both approaches can be used for braids with an index sequence or braids with a head/tail structure.

The resulting braids can be purified as is known in the art and as described for the two-step amplification method.

Although it is possible that each braid has a different size, it is more efficient when all braids have the same size, i.e. the same sequence length or number of nucleotides, respectively. Thus, it might be the case that for information to be stored in a braid not the whole length of the available sequence of a braid is needed, If the information does not fill the whole length of the braid, a defined short binary sequence can be added at the end of the information to be stored which acts as a universal tag. This universal tag has the function of a "stop codon", i.e. indicates where the information to be stored stops.

To decode the information of a data unit it is necessary to know the order of the braids and, thus the position of each braid in a row, i.e. if a braid is the first one, the second one, the $n^{th}$ one or the terminal one. To provide this information each braid comprises a semantic part. The semantic part can be an index type part, i.e. provide an index for each braid which indicates the order of the braids. Another option is to provide a structure for the braids of a data unit that allows this allocation. As one example, in the braids, the heads and tails can provide the information about the "position" of a braid, when the head string of a braid is identical to the tail string of the preceding braid, starting string and terminal string are unique. For this embodiment of a data unit, there will be one starting string, pairs of head and tail strings, where each pair can be used only once per data unit, center strings, where each center string can be used only once per braid, and one terminal string which can be used only once in a braid of a data unit.

Thus, in one embodiment strings and/or ropes are assembled to braids to provide a data system which comprises a head braid, at least one head/tail braid and a terminal braid, wherein in the head braid the head string is a starter string, that is present only once in the first braid but at no other end of a braid in the data unit, wherein a head/tail braid is a braid comprising a head string, a number of center strings and a tail string, each head string being identical to the tail string of a preceding or following braid thereby defining the order of the braids, and wherein in the terminal braid the tail string is a terminal string that is present only once in the terminal braid but at no other end of a braid in the data unit. A head braid in this case is the first braid having a starter string as first string. A head/tail braid is a braid for which the order is determined by a head/tail pair, wherein the head string is identical to the tail string of, depending on the definition, the preceding or following braid. A terminal braid is the last braid of a data unit, having as last string a unique terminal string. A center string is a string that is neither a head nor a tail string but is in the middle of a braid.

Each braid comprises an ordered linear arrangement of strings selected from the library, wherein the ordered linear arrangement determines the linear arrangement of a plurality of strings combined in each braid. The string in the first position is the string that is the first sequence starting from the 5' end of the braid. Although it is possible that each braid comprises different number of strings, it is more efficient when each comprises the same number of strings. The number of strings within a braid is variable and can be chosen depending on the length of a string, the amount of information to be stored etc.

The maximal number of braids that can be used per data unit depends on the system used for identification. In the case where head/tail pairs are used for determining the order of braids, the number of strings/available permutations within a library is n minus 1. If for example in this case a library comprises 6 different strings, then up to 5 different braids can be made. There are three types of braids, wherein one type of braids is the first or starting braid, one type of braids is the last or terminal braid, and one type of braids is a center or internal braid.

In one embodiment of the present invention, a data unit of braids with a specific structure are used, with a oligonucleotide in the first position starting from the 5' end in the first polynucleotide, which is also called the head string of the first braid, which is different from the head string in any one of the other types of braids. Therefore the first braid is identifiable from the other braids by its unique starting string, it is also referred to as head braid. Another part of the data unit in this embodiment is an oligonucleotide in the last position starting from the 5' end, which is also called tail string of the braid. The tail string of the last braid is unique. The braid comprising this unique last string is also referred to as terminal braid. The other braids of a data unit of this embodiment comprise a head string, a tail string and an even number of inner strings, wherein each head string of a braid is identical to the tail string of the preceding braid, thereby defining the order of the braids. Therefore internal braids are identifiable by having head and tail strings which correspond to tail and head strings of further braids. The order of the internal strings is identifiable by matching the last string of a braid to the first string of the subsequent braid. The last string of the last braid of the plurality of braids is different from the first string in any one of the other types of braids. Therefore the terminal braid is identifiable by its unique last string, and by a first string which matches the last string of the last internal braid. The first string in each braid is unique. Furthermore, each string only occurs once per braid.

Strings, ropes, or braids can be amplified as is known to the skilled person. One possibility is an amplification reaction that results in rope formation which is carried out for each combination of strings necessary to assemble the braids as defined by the encoding algorithm. If a braid has been defined as having the order of strings 4-1-13-12-8-10, for example, then the following ropes have to be obtained: 4-1; 1-13, 13-12, 12-8, and 8-10.

Therefore the rope amplification is repeated with all combinations of primer pairs until all combinations of double-stranded oligonucleotides present in the order determined in the encoding algorithm have been obtained.

After all necessary ropes have been obtained, the braids are assembled by pooling the ropes that have to be present in one braid as determined by the encoding algorithm separately for each braid. The mixture of ropes is then amplified via PCR to obtain the completed braids. The ropes act as primers and templates for the polymerase. The end-standing ropes, i.e. the rope comprising the first string of the braid, and the rope comprising the last string of the braid should be overrepresented in the pool to facilitate the desired braid assembly. The amplification is carried out until the braid is complete. Intermediate products comprising only parts of the braid will be present in a small amount.

In one embodiment braids are marked by using an index sequence which can be the starting sequence or the terminal sequence of a braid. The index sequence can be relatively short, depending on the length of the index number, for example 2 to 20 nucleotides, such as 3 to 5 nucleotides, it can comprise one "index string" or more "index strings" such as 2 or 3 index strings . The index sequence does not contain any data, but is used only to store the index of the braid. One advantage of using an index sequence is, that there are no restrictions with regard to strings occurring as first or last string, as no pairing of tail and head is necessary. Moreover, using an index sequence for allocating an index to a braid allows to identify any braid in a data unit separately, without having to sequence all other braids. This allows to store a huge amount of braids in a single well. Moreover, the amount of data that can be stored inside a single braid is preferably constant. This makes the method less complex and more efficient.

The completed braid can be isolated and/or purified by methods as known in the art to remove the intermediate products. This can for example be done by size-separation on a gel, followed by extraction from the gel as is known in the art, or by trough size-separation DNA-binding membrane columns, as is known in the art.

The present invention also comprises a method of accessing the data information stored in the nucleic acid according to the method of the invention by sequencing the polynucleotides and assembling the information based on the encoding information.

The amplification reactions used in the method of the present invention can be any amplification method suitable for the purpose of assembling ropes, or braids.

The same applies to the sequencing reactions used to retrieve information stored in the nucleic acid according to the present invention.

## BRIEF DESCRIPTION OF THE DRAWINGS

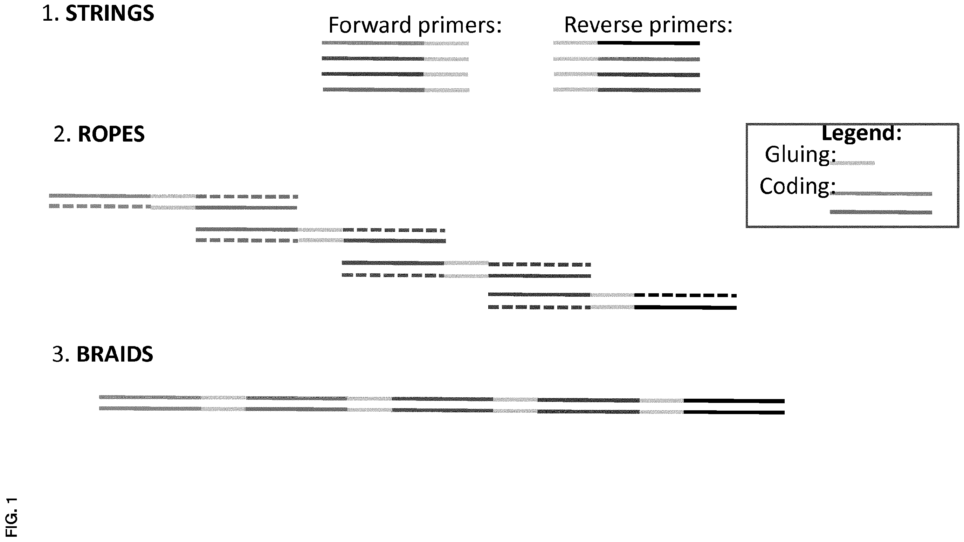

Figure 1 shows the experimental design and the nomenclature used in one embodiment of the method of the present invention. Step 1 shows the STRINGS, which are single-stranded oligonucleotides comprising a unique coding sequence and a gluing sequence (yellow). These strings are used as primers. Each string is present as forward primer and as reverse primer. Step 2 shows the rope formation using a pair of forward and reverse primers by PCR amplification. Two strings of choice (determined by the encoding order set by the encoding algorithm) are annealed via the gluing sequence, elongated and PCRed to give ropes, each of which consists of two coding sequences. Step 3 shows the braid formation. Braids are units that comprise a number of data bearing sequences, such as strings or ropes (5 strings in the example shown in Figure 1). The braids are obtained by assembling strings and/or ropes, for example by mixing equivolume amount of ropes and subjecting them to PCR using an excess (e.g. 10 times more) of the end ropes as primers.

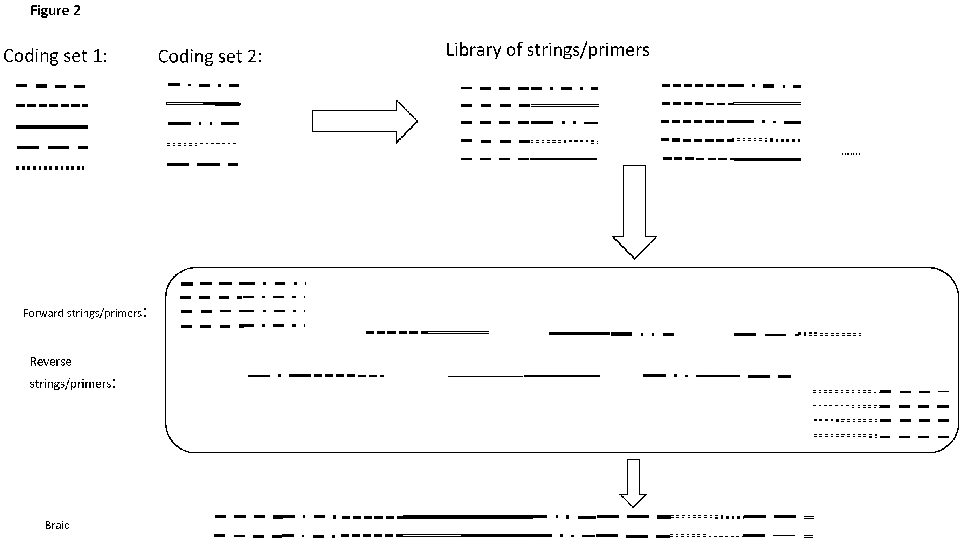

Figure 2 shows the experimental design used in another embodiment of the method of the present invention. For preparing a library of ropes, two sets of data bearing units - Coding set 1 and Coding set 2, wherein both sets have the same number of distinct oligonucleotides of predetermined length, are provided. Ropes can be created as Cartesian products of the two sets to provide a library of strings/primers as plurality of ropes, only some of which are shown as an example. Those ropes are combined by PCR assembly and amplification in a single step with "end primers" in surplus". The final product obtained is a braid comprising strings in the correct order.

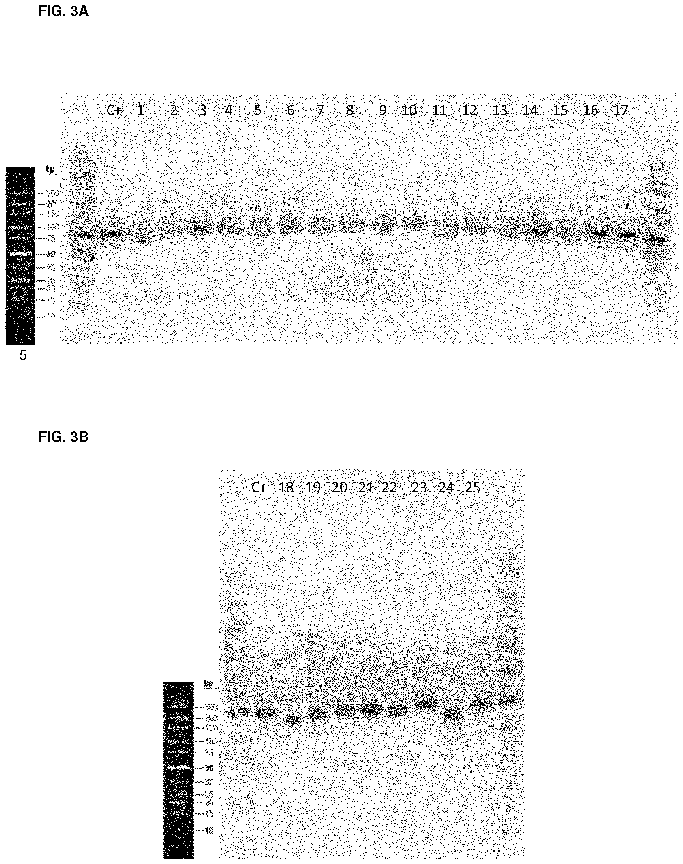

Figure 3 shows a gel image of the Ropes in which the word "BIOSISTEMIKA" is to be encoded. Four μl of each PCR product is loaded. On the left-hand side to the gel image, the O'GeneRuler Ultra Low Range DNA Ladder is denoted. Starting from left to right, the first lane contains 200 ng of the DNA ladder and the second lane contains -control 44 bp rope DNA product followed by first 17 ropes (Figure 3A) denoted in the Table 3, and followed by the last eight ropes denoted in the Table 3 (Figure 3B).

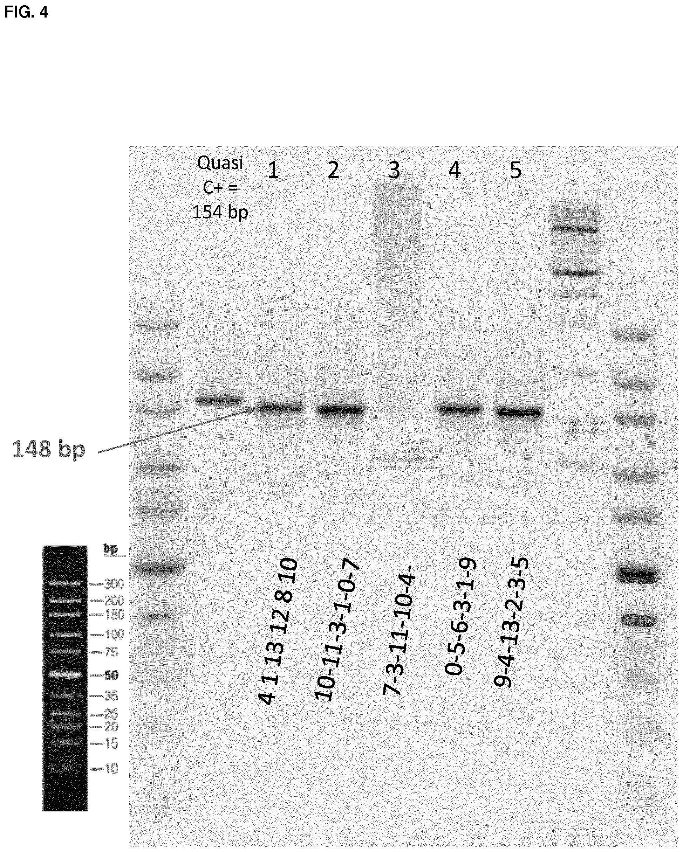

Figure 4 shows a gel image of the five Braids in which "BIOSISTEMIKA" is encoded. Four μl of each PCR product is loaded. On the left-hand side to the gel image, the O'GeneRuler Ultra Low Range DNA Ladder is denoted. Starting from left to right, the first lane contains 200 ng of the DNA ladder and the second lane contains quasi-control 154 bp DNA product followed by five Braids: 4 1 13 12 8 10, 10 11 3 1 0 7, 7 3 11 10 4 0, 0 5 6 3 1 9 and 9 4 13 2 3 5 and 750 ng of Quick-Load® 100 bp DNA Ladder and 200 ng of O'GeneRuler Ultra Low Range DNA Ladder.

In the following a method for preparing a coding data unit is outlined. This is only exemplary to more clearly show how ropes and braids can be obtained and how a data unit can be assembled.

The decoding process works in the same way, just in reverse. The detected permutation is enumerated, the number of available permutations counted and then the enumeration converted to the correct number of bits.

In this example a 2 letter redundancy is used meaning that up to two errors in the read coding sequences can be detected and corrected. This is achieved by using a Levenshtein or Hamming distance of 5, meaning that at least 5 (single base) changes are required to turn a used coding sequence into another used coding sequence. If there are no more than 2 errors, the resulting sequence will still be closer to its original state than any other used coding sequence.

Partial permutation enumeration

Throughout the text the term 'enumerating' partial permutation is used. This means that a number is assigned to each possible partial permutation, in a certain order. Each string has an allocated number. Both numbers are compared and when they match, the string is used.

The system of enumerating them is similar to a regular numerical system, but instead of the factors that are multiplied the digs with being powers of the base of the numerical system, they are the partial permutation numbers of the available strings.

The following is an example for enumeration:

A certain number of strings is available (n). These strings must be in an ordered list and carry an index.

A certain partial permutation having an integer is matched with the list of those strings (m strings) and the string with the correct integer is chosen. This is done by choosing the first string and removing it from the list (since strings cannot repeat in a permutation). The second string is then chosen from this now shortened list and so on. This is repeated until m strings have been chosen.

The index (in the remaining list) of the string is marked and chosen at each step with $o_i$. The index list starts with 0.

The enumeration of the partial permutation obtained this way is:

$$\sum_{i=1}^{m} \left( o_i * \prod_{j=n-m+1}^{n-i} j \right)$$

It is assumed that the following strings are available: [0,1,2,3,4,5,6,7,8,9]

This results in the following partial permutation: 1 0 4

a) First symbol

1 is the second symbol in the available list, giving an o1 of 1.
The product results in 72 (9*8).
This means that this symbol supplies the value of 72 (72*1)

b) Second symbol

0 is the first symbol in the available list, giving an o2 of 0.
The product results in 8.
This means that this symbol supplies the value 0 (8*0)

c) Third symbol

4 is the third symbol in the available list ([2,3,4,5,6,7,8,9]), giving an o3 of 2.
The product results in 1.
This means that this symbol supplies the value of 2 (1*2)
The sum of these values gives the partial permutation enumeration of 74.
The invention is in the following further explained by describing some examples.

**EXAMPLES**

**Example 1- Proof of concept encoding the word "BIOSISTEMIKA"**

In a first step a set of DNA sequences that were used as strings in the encoding system of the present invention was defined. The strings were chosen in a way that fulfilled a number of conditions:

a) Sufficient Levenshtein or Hamming distance between each pair of sequences, making it possible to detect and correct errors
b) A GC content between 0.5 and 0.55
c) Exactly two G/C bases in the last 5 bases
d) No more than two identical bases in a row, and no more than 2 of such repeats in the sequence.

In this example, sequences with a length of 18 nucleotides were used.
To encode the word "BIOSISTEMIKA" in DNA, a set of 14 forward and 14 reverse primers (standard desalting purification) were designed as set forth in Table 1. The primer sequences should satisfy the premises described above.

**Table 1.** Set of primers used for encoding "BIOSISTEMIKA" in DNA. Coding part is denoted in capital letters whereas gluing part is denoted in lowercase.

| Name | Forward primer sequence | Name | Reverse primer sequence |
|------|------------------------|------|------------------------|
| **C8G 0F** | GCAATTGATGGCGGTAGAgcgacaga SEQ ID NO:1 | **C8G 0R** | TCTACCGCCATCAATTGCtctgtcgc SEQ ID NO:2 |
| **C8G 1F** | TCTGCAAGCTGGAGTAGAgcgacaga SEQ ID NO:3 | **C8G 1R** | TCTACTCCAGCTTGCAGAtctgtcgc SEQ ID NO:4 |
| **C8G 2F** | TAGGATACGCCACACACTgcgacaga SEQ ID NO:5 | **C8G 2R** | AGTGTGTGGCGTATCCTAtctgtcgc SEQ ID NO:6 |
| **C8G 3F** | TAACGTCGGCTACTCACAgcgacaga SEQ ID NO:7 | **C8G 3R** | TGTGAGTAGCCGACGTTAtctgtcgc SEQ ID NO:8 |
| **C8G 4F** | TCGTGTTAGTCCGTCAGTgcgacaga SEQ ID NO:9 | **C8G 4R** | ACTGACGGACTAACACGAtctgtcgc SEQ ID NO:10 |
| **C8G 5F** | AACACTCGTCTCGACCATgcgacaga SEQ ID NO:11 | **C8G 5R** | ATGGTCGAGACGAGTGTTtctgtcgc SEQ ID NO:12 |

(continued)

| Name | Forward primer sequence | Name | Reverse primer sequence |
|---|---|---|---|
| C8G 6F | TCTATGCGACCACTACGAgcgacaga SEQ ID NO:13 | C8G 6R | TCGTAGTGGTCGCATAGAtctgtcgc SEQ ID NO:14 |
| C8G 7F | TGACAGAGCGTCTATCGTgcgacaga SEQ ID NO:15 | C8G 7R | ACGATAGACGCTCTGTCAtctgtcgc SEQ ID NO:16 |
| C8G 8F | ATGGCTATCGCTGATTGCgcgacaga SEQ ID NO:17 | C8G 8R | GCAATCAGCGATAGCCATtctgtcgc SEQ ID NO:18 |
| C8G 9F | TCCTGCGCTTATCAGAGTgcgacaga SEQ ID NO:19 | C8G 9R | ACTCTGATAAGCGCAGGAtctgtcgc SEQ ID NO:20 |
| C8G 10F | TCGCTAGAAGAGCAGAGTgcgacaga SEQ ID NO:21 | C8G 10R | ACTCTGCTCTTCTAGCGAtctgtcgc SEQ ID NO:22 |
| C8G 11F | AGTGTCCAGGATTGCATGgcgacaga SEQ ID NO:23 | C8G 11R | CATGCAATCCTGGACACTtctgtcgc SEQ ID NO:24 |
| C8G 12F | ACCGATACACGTCGTACAgcgacaga SEQ ID NO:25 | C8G 12R | TGTACGACGTGTATCGGTtctgtcgc SEQ ID NO:26 |
| C8G 13F | GTGCATGCATCACGATGAgcgacaga SEQ ID NO:27 | C8G 13R | TCATCGTGATGCATGCACtctgtcgc SEQ ID NO:28 |

In a second step the ASCII text "BIOSISTEMIKA" is converted into binary with 7 bit-per-letter encoding resulting in the following binary sequence:
1000010100100110011111010011100100110100111010100100010110011011001001 10010111000001

The starting binary has a length of 84 bits.

To convert from a number to a permutation, the permutations have to be enumerated. This is done by assigning a value to the 'offset' at each 'spot' of the permutation (the first choice is the first spot). The offset is the index of the choice that is made - picking the first choice that is available means an offset of 0, the second choice an offset of 1. The value of the offset is the number of possible partial permutations of all subsequent spots.

**Encoding 3 bits (100, 4), with options 0 1 2 3 4 5 6 7 8 9 10 11 12 13 and 1 places, which encodes to 4**

Here there are enough options to encode the first 3 bits. This is because there are 14 options (number of different strings), which is below 16 (2^4), but above (2^3). The value of these bits when converted to decimal is 4. Since this is encoded in only one 'string', the 5th option available (remember, 0 would be a valid option) has to be picked. Therefore the string with the assigned index "4" is picked.

**Encoding 14 bits (00101001001100, 2636), with options 0 1 2 3 5 6 7 8 9 10 11 12 13 and 4 places, which encodes to 1 13 12 8**

Here the situation is more complex, since the data in a sequence of 4 strings is encoded. There are a few more options (13 for the first spot, 12 for the second..., 13*12*11*10 in total, which is 17160, which translates into 14 bits (above 16384, but below 32768).

The enumeration of a permutation is calculated as follows. The 4th spot in the permutation will have a total of 10 options, and the value of an offset here will be 1. The 3rd spot will have 11 options, with the value of an offset here 10.

The 2nd spot will have 12 options, with the value of an offset here 110 (11*10).

The 1 st spot will have 13 options, with the value of an offset here 1320 (12*11*10).

The value 2636 has to be converted into a linear combination of these values, which turns out to be 1320 * 1 + 110 * 11 + 10 * 10 + 1 * 6

The offsets are: 1, 11, 10, 6. The offset value determines, which of the available options for strings is picked. As the first option initially had the index 0, the offset value +1 is the number of the choice.

So for the first sequence the second one (offset value 1 + 1 = 2) of the available ones is picked, which is the string with the assigned index 1.

For the second sequence the 12th choice is picked. As 1 is no longer available here, the string with the assigned index 13 is picked.

For the third sequence the 11th choice is picked. 1 and 13 are no longer available, the string with the assigned index 12 is picked

And for the fourth sequence the 7th choice is picked, which is the string with the assigned index 8.

Therefore the first braid is composed of the following internal strings:

1-13-12-8

This way of encoding is the same for the center of all the other braids. There is a special situation that arises for the endings though - their options are not just restricted by the strings used inside their own strings, but also by the endings of all other braids (and the beginning of the first one). The encoding of the end is 'increased' by a value of 1 for each other end that has already been used and could still be generated by the options available and would have a value lower and equal than the value that we are trying to encode.

**Encoding 2 bits (01, 1), with options 0 1 5 6 7 8 10 11 12 and 1 places, which encodes to 5**

Normally, this would be encoded as 1 (the second option). However, since 0 has already been used as the ending of the third braid, the value to be encoded is increased to 2, which means we use the third option instead (5).

Therefore the encoding algorithm defines the braid compositions as follows. BIOSISTEMIKA = 100001010010011001111101001110010011010011101010010001011000001 01101100100110010111000001

**Start of first braid**

Encoding 3 bits (100, 4), with options 0 1 2 3 4 5 6 7 8 9 10 11 12 13 and 1 places, which encodes to 4

**Center of braid 1**

Encoding 14 bits (00101001001100, 2636), with options 0 1 2 3 5 6 7 8 9 10 11 12 13 and 4 places, which encodes to 1 13 12 8

**Ending of braid 1**

Encoding 3 bits (111, 7), with options 0 2 3 5 6 7 9 10 11 and 1 places, which encodes to 10

**Center of braid 2**

Encoding 14 bits (11010011100100, 13540), with options 0 1 2 3 4 5 6 7 8 9 11 12 13 and 4 places, which encodes to 11 3 1 0

**Ending of braid 2**

Encoding 2 bits (11, 3), with options 2 4 5 6 7 8 9 12 13 and 1 places, which encodes to 7

**Center of braid 3**

Encoding 14 bits (01001110101001, 5033), with options 0 1 2 3 4 5 6 8 9 10 11 12 13 and 4 places, which encodes to 3 11 10 4

**Ending of braid 3**

Encoding 2 bits (00, 0), with options 0 1 2 5 6 8 9 12 13 and 1 places, which encodes to 0

**Center of braid 4**

Encoding 14 bits (01011001101100, 5740), with options 1 2 3 4 5 6 7 8 9 10 11 12 13 and 4 places, which encodes to 5 6 3 1

**Ending of braid 4**

Encoding 2 bits (10, 2), with options 2 4 7 8 9 10 11 12 13 and 1 places, which encodes to 9

**Center of braid 5**

Encoding 14 bits (01100101110000, 6512), with options 0 1 2 3 4 5 6 7 8 10 11 12 13 and 4 places, which encodes to 4 13 2 3

**Ending of braid 5**

Encoding 2 bits (01, 1), with options 0 1 5 6 7 8 10 11 12 and 1 places, which encodes to 5

First gluing data: 3

Decoding braid 1

Decoding braid 2

Decoding braid 3

Decoding braid 4

Decoding braid 5

Final binary, length 84

Therefore BIOSISTEMIKA is encoded as:

4 1 13 12 8 10

10 11 3 1 0 7

7 3 11 10 40

0 5 6 3 1 9

9 4 13 2 3 5

Meaning that, for encoding "BIOSISTEMIKA" into DNA, 5 different braids are needed, each of which comprising 6 coding sequences (see **Table 2**).

Table 2. "BIOSISTEMIKA" encoding syntax: coding sequences denoted as numbers which overlap between different braids are denoted in **bold.**

| Braid annotation (by the order) | Coding symbols present in a particular braid |
|---|---|
| 1 | 4 1 13 12 8 **10** |
| 2 | **10** 11 3 1 0 **7** |
| 3 | **7** 3 11 10 4 **0** |
| 4 | **0** 5 6 3 1 **9** |
| 5 | **9** 4 1 3 2 3 5 |

The encoding algorithm provides the blueprint for the following assembly of the braids via PCR amplification.
Separate reactions were made by mixing forward and reverse primer pairs according to Table 3. Each reaction tube corresponds to a separate rope.

**Table 3.** PCR reactions giving rise to the ropes.

| Tube | Rope annotation | Forward primer | Reverse primer |
|------|-----------------|----------------|----------------|
| 1 | 4-1 | C8G 4F | C8G 1R |
| 2 | 1-13 | C8G 1F | C8G 13R |
| 3 | 13-12 | C8G 13F | C8G 12R |
| 4 | 12-8 | C8G 12F | C8G 8R |
| 5 | 8-10 | C8G 8F | C8G 10R |
| 6 | 10-11 | C8G 10F | C8G 11R |
| 7 | 11-3 | C8G 11F | C8G 3R |
| 8 | 3-1 | C8G 3F | C8G 1R |
| 9 | 1-0 | C8G 1F | C8G 0R |
| 10 | 0-7 | C8G 0F | C8G 7R |
| 11 | 7-3 | C8G 7F | C8G 3R |
| 12 | 3-11 | C8G 3F | C8G 11R |
| 13 | 11-10 | C8G 11F | C8G 10R |
| 14 | 10-4 | C8G 10F | C8G 4R |
| 15 | 4-0 | C8G 4F | C8G 0R |
| 16 | 0-5 | C8G 0F | C8G 5R |
| 17 | 5-6 | C8G 5F | C8G 6R |
| 18 | 6-3 | C8G 6F | C8G 3R |
| 19 | 3-1 | C8G 3F | C8G 1R |
| 20 | 1-9 | C8G 1F | C8G 9R |
| 21 | 9-4 | C8G 9F | C8G 4R |
| 22 | 4-13 | C8G 4F | C8G 13R |
| 23 | 13-2 | C8G 13F | C8G 2R |
| 24 | 2-3 | C8G 2F | C8G 3R |
| 25 | 3-5 | C8G 3F | C8G 5R |

Each of the PCR reactions was performed in 20 $\mu$l volume using the following final concentrations: 200 $\mu$M of each of the dNTPs, 600 nM each of the forward and revers primers and 1.2 U/100 $\mu$l reaction of Deep Vent DNA polymerase (NEB, 2U/ $\mu$l). The thermal profile was: 30 sec at 30°C (initial annealing), 1 min at 72°C (initial elongation) followed by 20 cycles of 15 sec at 95°C, 15 sec at 30°C and 15 sec at 72°C, ending with 5 min elongation step at 72°C.

The ideal thermal profile would imply using 28°C in the annealing steps, since it is the theoretical annealing of the 8-nt long gluing sequence, but some thermal cyclers do not allow for this low temperature to be programmed during cycling. It was surprisingly realized that the assembly of ropes is not very susceptible to the annealing temperature because the ropes have worked out well even when 50°C annealing temperature was used. Using 50°C annealing is recommended in this case since it is time-saving: it takes less time for the thermal cycler to heat or cool because the temperature difference between the PCR steps (95/72°C) is smaller for 50°C annealing in comparison to 30°C annealing.

All the reactions were assembled on ice and quickly transferred to a thermocycler (Bio Rad T100) preheated to the denaturation temperature (95°C). All components are mixed and centrifuged prior to use. It is important to add Deep Vent DNA polymerase last in order to prevent any primer degradation caused by its 3'→ 5' exonuclease activity.

Reaction products should be checked in 3% agarose gel made by using 1x lithium borate (LB) buffer (both as casting and running buffer) pre-stained with SybrGreen (or equivalent) following the user manual (1:10 000 dilution) as shown in **Figure 3A** and **Figure 3B.**

In a second PCR amplification the braids were assembled.

Three $\mu$l of each of the five ropes needed for constructing a particular braid were mixed and centrifuged in separate tubes (corresponding to five different braids) according to the **Table 4.**

**Table 4.** Equivolume mix of ropes for obtaining the template for constructing braids.

| Tube | Mix of ropes to form braids (templates) | First rope (primer 1) | Last rope (primer 2) |
|---|---|---|---|
| 1 | **4-1** + 1-13 + 13-12 + 12-8 + **8-10** | **4-1** | **8-10** |
| 2 | **10-11** + 11-3 + 3-1 + 1-0 + **0-7** | **10-11** | **0-7** |
| 3 | **7-3** + 3-11 + 11-10 + 10-4 + **4-0** | **7-3** | **4-0** |
| 4 | **0-5** + 5-6 + 6-3 + 3-1 + **1-9** | **0-5** | **1-9** |
| 5 | **9-4** + 4-13 + 13-2 + 2-3 + **3-5** | **9-4** | **3-5** |

Each of the five PCR reactions was performed in 20 $\mu$l volume using the following final concentrations: 200 $\mu$M of each of the dNTPs, 0.2 v/v of each of the end ropes per braid, 0.08 v/v of the mix of ropes of a particular braid and 1.2 U/100 $\mu$l reaction of Deep Vent DNA polymerase (NEB). The thermal profile was: 2 min at 95°C (initial denaturation), followed by 35 cycles of 15 sec at 95°C, 15 sec at 55°C and 15 sec at 72°C, ending with 5 min elongation step at 72°C.

Note that in this reaction, the mix of ropes acts as a template and it is present in the reaction in 0.08 v/v amount (1.6 $\mu$l of the rope mix per 20 $\mu$l reaction volume), whereas there are 2.5 times more of each of the two "end ropes" which act as primers, thus, are added in the reaction surplus. End ropes are the first and the last rope (**Table 4**) in a particular braid and each makes 20% of the reaction (0.2 v/v or 4 $\mu$l of each per 20 $\mu$l reaction).

The template mix of ropes also contains end ropes meaning that, stoichiometrically, each of the end ropes are present in the reaction in 0.2+(0.08/5)=0.216 v/v (or 21.6%). In other words, 43.2% reaction consists of the both end ropes. In that manner, the reaction is biased towards obtaining primarily the correct braid product. Reaction intermediates are present in much smaller amount, as revealed in the **Figure 4** as extra bands of the sizes greater or less than 148 bp.

In addition, no traditional purification steps are involved in this two-step assembly because they are unnecessary for the setup of the invention and due to economic reasons. Anyhow, the primers, dNTPs and enzyme that are not used in the previous reaction 1 (rope formation), are transferred and utilized in the reaction 2 (braid formation).

Prior to sequencing, braids were purified using MinElute PCR Purification Kit (Quiagen), following the user manual (membrane cut-off = 70 bp - 4 kb). All the centrifugations were done at 17.800 x g. DNA was eluted in 20 $\mu$l $H_2O$ which was standing for 5 minutes on the membrane prior to 2 minutes centrifugation.

To prove the correct assembly of ropes into braids, the braids were sent to a Sanger sequencing company. Primers that form 5' and 3' ends of a particular braid were used both to sequence that particular braid. For example, in the case of braid 1: 4 1 13 12 8 10, C8G 4F and C8G 10 R were used (see **Table 1**). Forward primer read the last four Strings (e.g. 13 12 8 10) towards the 3' end, whereas the reverse primer read the first four Strings towards the 5' end (4 1 13 12). This was done for each of the five braids. Chromatogram data was extracted using SnapGene Viewer v. 4.1.9. All reads were readable between approx. 25-130 bases. Sequences were analyzed using EMBOSS Needle: https://www.ebi.ac.uk/Tools/psa/emboss_needle/ using default parameters.

Unspecific products were not discovered by Sanger sequencing. If there were unspecific products which might interfere with NGS technology this could be easily circumvented by post-read processing ie, taking in count solely the read of the correct size (148 bp).

**Example 2**

Multiple step assembly with gluing sequences is shown in the following scheme:

| Coding 1F | Gluing |
|---|---|

| Gluing | Coding 2 R |
|---|---|

\+

| Coding 2 F | Gluing |
|---|---|

| Gluing | Coding 3 R |
|---|---|

\+

| Coding 3 F | Gluing |
|---|---|

| Gluing | Coding 4 R |
|---|---|

AsAssembly and multiplication results in the braid:

| Coding 11 F | Coding 21 F |
|---|---|

| Coding 11 F | Coding 21 F | Coding 12 F | Coding 22 F | Coding 13 F | Coding 23 F |
|---|---|---|---|---|---|

| Coding 21 R | Coding 12 R | Coding 22 R | Coding 13 R | Coding 23 F | Coding 13 R |
|---|---|---|---|---|---|

| Coding 23 F | Coding 13 R |
|---|---|

**Claims**

1. Method of storing information in nucleic acid comprising

   a) processing units of information into permutation numbers by a reversible algorithm,
   b) providing a library of *n* distinct oligonucleotide strings of predetermined length in a fixed order, wherein *n* is a positive integer, wherein each distinct oligonucleotide string is associated with a distinct index indicating the ordinal position,
   c) assembling distinct oligonucleotide strings to create strands comprising at least two oligonucleotide strings, wherein each oligonucleotide string's ordinal position matches with a permutation number obtained in step a), wherein each strand comprises at least a data bearing part and a semantic part, wherein the semantic part is to allocate orientation and/or order to a strand.

2. Method of storing information in nucleic acid according to claim 1 wherein in step a) starting information is digital data.

3. Method of storing information in nucleic acid according to claim 1 or claim 2 wherein digital data are chopped to smaller pieces, read as an integer and are processed to permutation numbers by using partial permutation enumeration.

4. Method of storing information in nucleic acid according to any of the preceding claims, wherein distinct strings are assembled such that two strings are combined to a rope, and ropes are assembled to build braids.

5. Method of storing information in nucleic acid according to any of the preceding claims wherein in step b) a library is provided which comprises two sets of distinct strings and wherein ropes are created as Cartesian products of the two sets, wherein each rope comprises two distinct strings and optionally a gluing part.

6. Method of storing information in nucleic acid according to any of the preceding claims wherein in step b) a library is provided which comprises two sets of distinct strings and wherein braids are created as an alternating sequence of strings of both sets, wherein in the braid a string from one of the sets is followed by a string of the other set and the sequence of the strings from each of those sets represents a partial permutation.

7. Method of storing information in nucleic acid according to any of the preceding claims wherein in step c) strings and/or ropes are assembled to braids to provide a data system which comprises a head braid, at least one head/tail braid and a terminal braid, wherein in the head braid the head string is a starter string, that is present only once in the first braid but at no other end of a braid in the data unit, wherein a head/tail braid is a braid comprising at least one head string, a number of center strings and a tail string, each head string being identical to the tail string of the preceding braid thereby defining the order of the braids, and wherein in the terminal braid the tail string is a terminal string that is present only once in the terminal braid but at no other end of a braid in the data unit.

8. Method of storing information in nucleic acid according to any of the preceding claims wherein in step c) a plurality of braids is assembled each comprising an ordered linear arrangement of single-stranded oligonucleotide strings selected from a library of strings, wherein the ordered linear arrangement determines the linear arrangement of a plurality of single-stranded oligonucleotide strings combined in each braid, wherein the plurality of braids comprises three types of strings, wherein one type of strings is a head string, one type of strings is a terminal string, and one type of strings is center string, wherein the single-stranded head string(s) starting from the 5' end in the first braid is different from the single-stranded head string(s) starting from the 5' end in any one of the other braids, and wherein the single-stranded tail string(s) starting from the 5' end is the same as the single-stranded head string(s) starting from the 5' end in a second braid, wherein the single-stranded tail string(s) starting from the 5' end in the last braid of the plurality of braids is different from the single-stranded head string(s) starting from the 5' end in any one of the other braids, and wherein the single-stranded head string(s) starting from the 5' end is unique for each braid; and wherein the single-stranded terminal string occurs only once per data unit; and assembling the plurality of strings or ropes by PCR amplification using single-stranded strings or ropes as primers.

9. Method of storing information in nucleic acid according to any of the preceding claims, wherein the library of single-stranded oligonucleotides comprises a plurality of pairs of single-stranded oligonucleotides, wherein each pair comprises a forward and a reverse primer, wherein the forward primer comprises a first single-stranded oligonucleotide comprising a first coding sequence at its 5' end and a first gluing sequence at its 3' end, and wherein the reverse primer comprides a second coding sequence et its 5' end and a second gluing sequence at its 3' end, wherein the second coding sequence is complementary and inverse to the first coding sequence, and wherein the second gluing sequence is complementary and inverse to the first gluing sequence, and wherein the first gluing sequence in each pair of single-stranded oligonucleotides is identical, and wherein each coding sequence is unique.

10. Method of storing information in nucleic acid according to any of the preceding claims comprising a step of annealing a forward primer of a first pair of single-stranded oligonucleotide strings to a reverse primer of a second pair of single-stranded oligonucleotide strings, extending both the reverse and forward primers to obtain a double-stranded rope, wherein one strand comprises the coding sequence of the forward primer of the first pair at its 5' end, and the coding sequence of the forward primer of the second pair at its 3' end, wherein the gluing sequence is between the coding sequences, wherein the strings of the first and second pair have been selected to match with a permutation number obtained in step a).

11. Method of storing information in nucleic acid according to any of the preceding claims comprising repeating the step of claim 10 until a braid has been obtained, and/or isolating braids after assembly.

12. Method of storing information in nucleic acid according to any of the preceding claims wherein strands are assembled by pooling double-stranded strings separately for each strand and amplifying the mixture of strings to obtain the completed strands.

13. Method of storing information in nucleic acid according to any of the preceding claims wherein a library is provided which comprises a plurality of single-stranded ropes, each comprising two data bearing sequences, wherein the ropes have been obtained as cartesian products of two sets of single-stranded strings, wherein optionally braids are assembled by pooling single-stranded ropes each comprising two strings in an order as defined according to step a), and amplifying the mixture of ropes to obtain braids, wherein optionally a braid can be obtained by annealing ropes, wherein any rope has an overlapping part with another rope, wherein the overlapping part is a forward primer or a reverse primer, respectively, such that one string comprises a forward primer and a second string comprises a corresponding reverse primer.

14. Method of any of the preceding claims, wherein the sequence of the single-stranded string has a length of about 4 to about 500 nucleotides, and/or wherein the GC content is between 0.35 and 0.75; and/or wherein the Levenshtein distance or Hamming distance between each pair of coding sequences is at least 3; and/or wherein the number of G/C bases that are present in the last 5 to 10 bases of each coding sequence is predetermined, and/or wherein there are up to 3 identical bases in a row, and/or wherein the number of base repeats in each coding sequence is predetermined and/or wherein a gluing sequence has a length of about 4 to about 60 nucleotides.

15. Method of accessing the data information stored in the nucleic acid obtained according to any of the preceding claims by sequencing sequences of the data system and decoding the information by using the reverse algorithm.

FIG. 1

**Figure 2**

Coding set 1:     Coding set 2:                    Library of strings/primers

Forward strings/primers:

Reverse strings/primers:

Braid

FIG. 3A

FIG. 3B

**FIG. 4**

Europäisches
Patentamt

European
Patent Office

Office européen
des brevets

## EUROPEAN SEARCH REPORT

Application Number

EP 19 17 7466

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YAZDI S M HOSSEIN TABATABAEI ET AL: "DNA-Based Storage: Trends and Methods", IEEE TRANSACTIONS ON MOLECULAR, BIOLOGICAL AND MULTI-SCALE COMMUNICATIONS, IEEE, vol. 1, no. 3, 1 September 2015 (2015-09-01), pages 230-248, XP011605293, DOI: 10.1109/TMBMC.2016.2537305 [retrieved on 2016-04-04] * throughout, e.g. abstract, pg. 230 * | 1-15 | INV. G16B50/20 |
| X | WO 2013/178801 A2 (EUROPEAN MOLECULAR BIOLOGY LAB EMBL [DE]) 5 December 2013 (2013-12-05) * throughout, e.g. [0006]-[0016], [0016], [0027], [0039], Fig.2 * | 1-15 | |
| X | WO 2015/144858 A1 (THOMSON LICENSING [FR]) 1 October 2015 (2015-10-01) * abstract, pgs. 2-9, Figs. 1, 2 * | 1-15 | |
| X | MASAHITO YAMAMOTO ET AL: "Large-scale DNA memory based on the nested PCR", NATURAL COMPUTING, KLUWER ACADEMIC PUBLISHERS, DO, vol. 7, no. 3, 19 March 2008 (2008-03-19), pages 335-346, XP019612124, ISSN: 1572-9796 * throughout, e.g. abstract, Fig. 1 * | 1-15 | TECHNICAL FIELDS SEARCHED (IPC) G16B |

-/--

| The present search report has been drawn up for all claims | | |
|---|---|---|
| Place of search | Date of completion of the search | Examiner |
| Munich | 17 December 2019 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

Application Number

EP 19 17 7466

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAMES BORNHOLT ET AL: "A DNA-Based Archival Storage System", ARCHITECTURAL SUPPORT FOR PROGRAMMING LANGUAGES AND OPERATING SYSTEMS, ACM, 2 PENN PLAZA, SUITE 701 NEW YORK NY 10121-0701 USA, 25 March 2016 (2016-03-25), pages 637-649, XP058079901, DOI: 10.1145/2872362.2872397 ISBN: 978-1-4503-4091-5 * abstract, pgs. 637, 641-644 * | 1-15 | |
| X | S. M. HOSSEIN TABATABAEI YAZDI ET AL: "A Rewritable, Random-Access DNA-Based Storage System", SCIENTIFIC REPORTS, vol. 5, 18 September 2015 (2015-09-18), page 14138, XP055250233, DOI: 10.1038/srep14138 * throughout, e.g. pgs. 1, 2 * | 1-15 | |
| X | LEONID V. BYSTRYKH: "Generalized DNA Barcode Design Based on Hamming Codes", PLOS ONE, vol. 7, no. 5, 17 May 2012 (2012-05-17), page e36852, XP055560951, DOI: 10.1371/journal.pone.0036852 * throughout, e.g. abstract, pg. 1, Fig. 1 * | 1-15 | TECHNICAL FIELDS SEARCHED (IPC) |

-/--

The present search report has been drawn up for all claims

| Place of search | Date of completion of the search | Examiner |
|---|---|---|
| Munich | 17 December 2019 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 19 17 7466

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TILO BUSCHMANN ET AL: "Levenshtein error-correcting barcodes for multiplexed DNA sequencing", BMC BIOINFORMATICS, vol. 14, no. 1, 1 January 2013 (2013-01-01), page 272, XP055194297, ISSN: 1471-2105, DOI: 10.1186/1471-2105-14-272 * throughout, e.g. abstract, pgs 2-4 * ----- | 1-15 | |

|  |  |  | TECHNICAL FIELDS SEARCHED (IPC) |
|---|---|---|---|

The present search report has been drawn up for all claims

| Place of search | Date of completion of the search | Examiner |
|---|---|---|
| Munich | 17 December 2019 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 17 7466

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2019

| Patent document cited in search report | | Publication date | Patent family member(s) | | Publication date |
|---|---|---|---|---|---|
| WO 2013178801 | A2 | 05-12-2013 | AU | 2013269536 A1 | 18-12-2014 |
| | | | AU | 2018247323 A1 | 08-11-2018 |
| | | | CA | 2874540 A1 | 05-12-2013 |
| | | | CN | 104520864 A | 15-04-2015 |
| | | | CN | 107055468 A | 18-08-2017 |
| | | | DK | 2856375 T3 | 05-11-2018 |
| | | | EP | 2856375 A2 | 08-04-2015 |
| | | | EP | 3346404 A1 | 11-07-2018 |
| | | | ES | 2698609 T3 | 05-02-2019 |
| | | | HK | 1208937 A1 | 18-03-2016 |
| | | | IL | 235954 A | 28-02-2019 |
| | | | JP | 2015529864 A | 08-10-2015 |
| | | | JP | 2019023890 A | 14-02-2019 |
| | | | KR | 20150016572 A | 12-02-2015 |
| | | | RU | 2014152796 A | 27-07-2016 |
| | | | SG | 11201407818P A | 30-12-2014 |
| | | | US | 2015261664 A1 | 17-09-2015 |
| | | | US | 2019370164 A1 | 05-12-2019 |
| | | | WO | 2013178801 A2 | 05-12-2013 |
| WO 2015144858 | A1 | 01-10-2015 | EP | 3123376 A1 | 01-02-2017 |
| | | | US | 2017187390 A1 | 29-06-2017 |
| | | | WO | 2015144858 A1 | 01-10-2015 |

EPO FORM P0459

For more details about this annex : see Official Journal of the European Patent Office, No. 12/82